(19) Europäisches Patentamt
European Patent Office
Office européen des brevets

(11) Veröffentlichungsnummer: **0 320 727 B1**

# (12) EUROPÄISCHE PATENTSCHRIFT

(45) Veröffentlichungstag der Patentschrift: **02.01.92**

(51) Int. Cl.$^5$: **C07C 323/58**, C07C 319/02

(21) Anmeldenummer: **88120207.1**

(22) Anmeldetag: **03.12.88**

## (54) Verfahren zur Herstellung von Cysteamin-Säureadditionssalzen.

(30) Priorität: **12.12.87 DE 3742265**

(43) Veröffentlichungstag der Anmeldung:
**21.06.89 Patentblatt 89/25**

(45) Bekanntmachung des Hinweises auf die Patenterteilung:
**02.01.92 Patentblatt 92/01**

(84) Benannte Vertragsstaaten:
**CH DE FR GB IT LI NL**

(56) Entgegenhaltungen:
**DE-A- 3 025 461**

**CHEMICAL ABSTRACTS, Band 79, Nr. 19, 12. November 1973, Seite 354, Nr. 125805d, Columbus, Ohio, US; & JP-A-73 39 414**

(73) Patentinhaber: **BASF Aktiengesellschaft**
**Carl-Bosch-Strasse 38**
**W-6700 Ludwigshafen(DE)**

(72) Erfinder: **Hohmann, Andreas, Dr.**
**Sinsheimer Strasse 22**
**W-6700 Ludwigshafen(DE)**
Erfinder: **Reuther, Wolfgang, Dr.**
**Am Pferchelhang 16**
**W-6900 Heidelberg(DE)**
Erfinder: **Fikentscher, Rolf, Dr.**
**Von-Stephen-Strasse 27**
**W-6700 Ludwigshafen(DE)**
Erfinder: **Proll, Theo, Dr.**
**Mozartstrasse 30**
**W-6702 Bad Duerkheim(DE)**

EP 0 320 727 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von Cysteamin-Säureadditionssalzen durch Umsetzung von Aziridin mit einer anorganischen Schwefelverbindung der Oxidationsstufe -2 und mit einem Keton und anschließender saurer Hydrolyse des als Zwischenstufe gebildeten Thiazolidins, wobei man Aziridin und das Keton mit Ammoniumhydrogensulfid oder einem Metallhydrogensulfid unter Zugabe einer mittelstarken bis starken Säure bei einem pH-Wert, der größer oder gleich 8,5 ist, umsetzt.

Aus der DE-A-3 025 461 ist bekannt, 2-Aminoethylhydrogensulfat mit einem Keton zu einem Thiazolidinderivat umzusetzen und dieses anschließend durch saure Hydrolyse in ein Säureadditionssalz des Cysteamins überzuführen. Dieses Verfahren ist jedoch sehr aufwendig, da die Thiazolidinbildung teilweise im Autoklaven erfolgen und das resultierende Thiazolidin in allen Fällen nach seiner Aufarbeitung destillativ gereinigt werden muß. Außerdem ist in einer zusätzlichen Vorstufe der Schwefelsäurehalbester von 2-Aminoethanol herzustellen.

Die JP-A- 39 414/1973 beschreibt die Umsetzung von Aziridin mit einem Keton und Schwefelwasserstoff zu einem Thiazolidinderivat, das anschließend durch saure Hydrolyse in ein Säureadditionssalz des Cysteamins umgewandelt wird. Der dort beschriebene Syntheseweg zum Thiazolidinderivat ist auch aus Ann. Chem. Band 566, S. 210 f., 1950, und der DE-A-747 733 bekannt. Der Nachteil dieses Verfahrens ist in der Verwendung von Schwefelwasserstoff zu sehen. Aufgrund der hohen Toxizität von Schwefelwasserstoff ist bei dessen Anwendung ein hoher Sicherheitsaufwand zu betreiben.

Aufgabe der vorliegenden Erfindung war es nun, ein neues Verfahren zur Herstellung von Cysteamin-Säureadditionssalzen bereitzustellen, das in einfacher Weise und ohne großen apparativen oder sicherheitstechnischen Aufwand durchgeführt werden kann.

Es wurde gefunden, daß die Herstellung von Cysteamin-Säureadditionssalzen durch Umsetzung von Aziridin mit einer anorganischen Schwefelverbindung der Oxidationsstufe -2 und mit einem Keton der Formel I

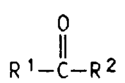 (I),

in der $R^1$ und $R^2$ gleich oder verschieden sind und unabhängig voneinander jeweils $C_1$-$C_6$-Alkyl oder $R^1$ und $R^2$ zusammen $C_4$-$C_6$-Alkylen bedeuten, und anschließender Hydrolyse des als Zwischenstufe gebildeten Thiazolidins der Formel II

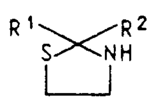 (II),

in der $R^1$ und $R^2$ jeweils die obengenannte Bedeutung besitzen, in Gegenwart einer Säure vorteilhaft gelingt, wenn man Aziridin und das Keton der Formel I mit Ammoniumhydrogensulfid oder einem Metallhydrogensulfid unter Zugabe einer mittelstarken bis starken Säure bei einer Temperatur von -10 bis +100 °C umsetzt und dabei einen pH-Wert, der größer oder gleich 8,5 ist, einhält.

$R^1$ und $R^2$ in Formel I bedeuten beispielsweise Methyl, Ethyl, Propyl, Isopropyl, Butyl, Isobutyl, sec-Butyl, Pentyl, Isopentyl, Neopentyl, tert-Pentyl, Hexyl oder 2-Methylpentyl.

Wenn $R^1$ und $R^2$ zusammen für $C_4$-$C_6$-Alkylen stehen, ist beispielsweise Tetramethylen, Pentamethylen, Hexamethylen, 2-Methyltetramethylen oder 2-oder 3-Methylpentamethylen als Rest zu nennen.

Vorzugsweise verwendet man Aceton, Methylethylketon oder Cyclohexanon als Keton der Formel I.

Bei den im erfindungsgemäßen Verfahren zu verwendenden Metallhydrogensulfiden handelt es sich beispielsweise um Alkali- oder Erdalkalihydrogensulfide, wie Lithium-, Natrium-, Kalium-, Magnesium- oder Calciumhydrogensulfid. Vorzugsweise verwendet man Ammoniumhydrogensulfid oder ein Alkalihydrogensulfid. Besonders bevorzugt ist Natrium- oder Kaliumhydrogensulfid.

Es ist natürlich auch möglich, von den entsprechenden Sulfiden auszugehen. Durch die erfindungsgemäße Zugabe einer mittelstarken bis starken Säure werden sie dann in die jeweiligen Hydrogensulfide übergeführt.

Als mittelstarke bis starke Säuren im erfindungsgemäßen Sinn sind in der Regel solche Säuren anzusehen, deren $pK_a$-Wert kleiner oder gleich 3,5 ist. Im allgemeinen beträgt der $pK_a$-Wert dabei -10 bis

+3,5.

Erfindungsgemäß können sowohl anorganische wie auch organische Säuren verwendet werden, wenn deren pKa-Wert die obengenannte Forderung erfüllt.

Solche Säuren sind beispielsweise Salzsäure, Bromwasserstoffsäure, Flußsäure, Schwefelsäure, Salpetersäure, Phosphorsäure, Trifluoressigsäure, Trichloressigsäure, Methansulfonsäure, Benzolsulfonsäure oder p-Toluolsulfonsäure.

Bevorzugt verwendet man eine starke anorganische Säure. Die Verwendung von Salzsäure ist besonders bevorzugt.

Das erfindungsgemäße Verfahren, das sowohl in kontinuierlicher als auch in diskontinuierlicher Arbeitsweise vorgenommen werden kann, wird zweckmäßig so durchgeführt, daß man das Keton I und das jeweilige Hydrogensulfid, das vorteilhaft in Form einer 10 bis 70 gew.%igen wäßrigen Lösung zur Anwendung kommt, vorlegt und zu dieser Mischung Aziridin und die mittelstarke bis starke Säure gibt. Die Zugabe von Aziridin und Säure kann zeitlich versetzt, d.h. zuerst erfolgt die Zugabe von Aziridin und dann die Zugabe der Säure, oder vorzugsweise gleichzeitg, aber räumlich getrennt voneinander erfolgen. Es ist auch möglich, Aziridin und Säure in abwechselnder Reihenfolge portionsweise zuzugeben.

Die Umsetzung erfolgt vorzugsweise unter atmosphärischem Druck oder aber auch unter leicht erhöhtem Druck (bis zu ca. 2 bar) und kann mit oder ohne Schutzgas vorgenommen werden. Vorzugsweise arbeitet man in Gegenwart eines Schutzgases. Als Schutzgas kommt z.B. Stickstoff oder Helium in Betracht.

Wichtig ist, daß während der Umsetzung ein pH-Wert, der größer oder gleich 8,5 ist und vorzugsweise 9 bis 12 beträgt, eingehalten wird.

Pro Mol Keton I benötigt man im allgemeinen 0,5 bis 1,5 Mol Aziridin, 0,5 bis 1,5 Äquivalent Hydrogensulfid und 0,5 bis 1,5 Äquivalent Säure.

Die Zeit, die für die Zugabe von Aziridin und Säure benötigt wird, beträgt im allgemeinen 0,5 bis 5 Stunden, vorzugsweise 1 bis 2 Stunden. Die Reaktionstemperatur beträgt -10 bis +100 °C, vorzugsweise 0 bis 60 °C und insbesondere 20 bis 40 °C.

Nach einer Nachrührphase von 0,5 bis 4 Stunden, die im obengenannten Temperaturbereich erfolgt, wird die entstandene organische Phase, die das Thiazolidin II enthält, von der wäßrigen Phase abgetrennt und zum Zweck der Hydrolyse mit Säure versetzt.

Als Säure kommt in diesem Fall zweckmäßig die gleiche Säure in Betracht, die bei der Umsetzung des Aziridins zugegeben wird. Es ist aber auch möglich, eine andere Säure zur Hydrolyse zu verwenden. In erster Linie richtet sich die Wahl der Säure in diesem Fall danach, welches Cysteamin-Säureadditionssalz man am Ende vorsieht. Die Verwendung von Salzsäure ist auch hier bevorzugt.

Man gibt in der Regel 1 bis 1,2 Äquivalent, vorzugsweise 1,05 bis 1,2 Äquivalent Säure, jeweils bezogen auf ein Äquivalent basischen Stickstoff in der organischen Phase zu und erhitzt das Reaktionsgemisch zum Sieden (ca. 70 bis 110 °C), wobei das Thiazolidin II hydrolysiert wird und das bei der Hydrolyse entstehende Keton I abdestilliert.

Nach einer Reaktionsdauer von 0,5 bis 4 Stunden ist die Hydrolyse beendet. Die erhaltene Lösung des Cysteamin-Säureadditionssalzes wird abgekühlt und bedarf keiner weiteren Reinigung. Es ist nun möglich, das Cysteamin-Salz vom Wasser zu befreien oder auch direkt in Lösung weiterzuverarbeiten. In manchen Fällen kann es auch von Vorteil sein, die wäßrige Lösung des Cysteamin-Säureadditionssalzes noch mit Aktivkohle zu behandeln.

Cysteamin-Hydrochlorid ist ein wertvolles Zwischenprodukt für die Herstellung der $H_2$-Blocker Cimetidin und Ranitidin.

Die folgenden Beispiele sollen die Erfindung näher erläutern.

Beispiel 1

420 g (3,0 mol) 40 gew.%ige wäßrige Natriumhydrogensulfid-Lösung und 238 g (3,3 mol) Ethylmethylketon wurden unter Stickstoff vorgelegt. Dazu ließ man unter Rühren gleichzeitig und kontinuierlich innerhalb von 1,5 Stunden aus getrennten Zulaufgefäßen 215 g (3,0 mol) 60 gew.%iges wäßriges Aziridin und 365 g (3,0 mol) 30 gew.%ige Salzsäure tropfen, wobei der pH-Wert von 8,5 nicht unterschritten wurde. Die Reaktionstemperatur bewegte sich zwischen 30 °C und 40 °C. Nach beendeter Zugabe wurde 1 Stunde bei 50 °C nachgerührt.

Die untere (wäßrige) Phase wurde abgetrennt und die obere (thiazolidinhaltige) Phase (390 g, 2,48 Äquivalent basischer Stickstoff = 83 % Ausbeute, bezogen auf Aziridin) mit 989 g (2,7 mol) 10 gew.%iger Salzsäure versetzt. Die Lösung wurde erhitzt und Ethylmethylketon/Wasser bis zu einer Übergangstemperatur von 100 °C abdestilliert. Die klare Reaktionslösung wurde eingeengt und 279 g (2,46 mol = 82 % d.

Th.) farbloses Cysteamin-Hydrochlorid mit einem Schmelzpunkt von 63 - 67° C isoliert.

| | | | | | | |
|---|---|---|---|---|---|---|
| $C_2H_8NSCl$<br>113,61 | Ber.:<br>Gef.: | C = 21,2<br>C = 21,2 | H = 7,0<br>H = 7,0 | N = ,3<br>N = ,3 | 12 S = ,2<br>12 S = ,2 | 28 Cl = 31,3<br>27 Cl = 31,3 |

SH-Gehalt (iodometrisch): 8,84 mmol/g (100 % d.Th.)

Umkristallisation aus Isopropanol lieferte Cysteamin-Hydrochlorid vom Schmelzpunkt 63 - 68° C.

Beispiel 2

336 g (3,0 mol) 50 gew.%ige wäßrige Natriumhydrogensulfid-Lösung und 238 g (3,3 mol) Ethylmethylketon wurden unter Stickstoff vorgelegt und das Gemisch mit 20 gew.%iger Salzsäure auf pH 9 eingestellt. Hierzu wurden 129 g (3,0 mol) Aziridin getropft. Dabei wurde der pH-Wert mit 20 gew.%iger Salzsäure zwischen 8,9 und 9,1 gehalten. Die Innentemperatur betrug 30 bis 40° C. Nach beendeter Zugabe wurde eine Stunde unter Rückfluß erhitzt, auf 25° C abgekühlt, mit Salzsäure auf pH 8,5 gestellt und die untere Phase abgetrennt. Der Gesamtverbrauch an 20 gew.%iger Salzsäure betrug 594 g, (3,3 mol).

Die thiazolidinhaltige Oberphase (375 g, 2,26 Äquivalent basischer Stickstoff = 75 % Ausbeute, bezogen auf Aziridin) wurde mit 913 g (2,5 mol) 10 gew.%iger Salzsäure versetzt, das freigesetzte Ethylmethylketon abdestilliert, die rohe Produktlösung über 20 g Aktivkohle filtriert und eingeengt.
Ausbeute Cysteamin-Hydrochlorid: 222 g (1,95 mol = 65 % d. Th.)
Fp.: 60 - 68° C (Umkrist. aus Isopropanol: 62 - 67° C)

| | | | | | | |
|---|---|---|---|---|---|---|
| $C_2H_8NSCl$<br>113,61 | Ber.:<br>Gef.: | C = 21,1<br>C = 20,8 | H = 7,0<br>H = 6,6 | N = ,3<br>N = ,1 | 12 S = ,2<br>12 S = ,8 | 28 Cl = 31,3<br>26 Cl = 31,8 |

SH-Gehalt (iodometrisch): 8,84 mmol/g (100 % d. Th.)

Beispiel 3

Analog Beispiel 2 wurde die Reaktion mit Cyclohexanon als Keton durchgeführt.

Verbrauch an 20 gew.%iger Salzsäure während der Umsetzung: 550 g (3,0 mol)
Thiazolidinhaltige Phase: 510 g (2,66 Äquivalent basischer Stickstoff = 89 % Ausbeute, bezogen auf Aziridin)

Die Hydrolyse von Thiazolidin wurde durch Zugabe von 1070 g (2,9 mol) 10 gew.%iger Salzsäure und anschließender azeotroper Destillation des Cyclohexanons erreicht. Die Rohproduktlösung wurde zweimal über Aktivkohle gefiltert.

Ausbeute Cysteamin-Hydrochlorid: 261 g (2,29 mol = 77 % d. Th.)
Fp.: 63 - 66° C (Umkristallisation aus Isopropanol: 60 - 67° C)

| | | | | | | |
|---|---|---|---|---|---|---|
| $C_2H_8NSCl$<br>113,61 | Ber.:<br>Gef.: | C = 21,1<br>C = 21,0 | H = 7,0<br>H = 7,1 | N = ,3<br>N = ,0 | 12 S = ,2<br>12 S = ,5 | 28 Cl = 31,3<br>26 Cl = 32,5 |

SH-Gehalt (iodometrisch): 8,70 mmol/g (99 % d. Th.)

Beispiel 4

Analog Beispiel 2 wurde die Reaktion mit Aceton als Keton durchgeführt.

Verbrauch an 20 gew.%iger Salzsäure während der Umsetzung: 662 g (3,6 mol)
Thiazolidinhaltige Phase: 237 g (1,52 Äquivalent basischer Stickstoff = 51 % Ausbeute, bezogen auf Aziridin)

Nach Zugabe von 603 g (1,7 mol) 10 gew.%iger Salzsäure wurde wie in Beispiel 2 aufgearbeitet.

Ausbeute Cysteamin-Hydrochlorid: 161,3 g (1,42 mol = 47 % d. Th.)
Fp.: 55 - 63° C (Umkristallisation aus Isopropanol: 55 - 67° C)

| | | | | | | |
|---|---|---|---|---|---|---|
| $C_2H_8NSCl$ | Ber.: | C = 21,1 | H = 7,0 | N = ,3 | 12 S = ,2 | 28 Cl = 31,3 |
| 113,61 | Gef.: | C = 22,5 | H = 7,4 | N = ,7 | 11 S = ,2 | 26 Cl = 30,7 |

SH-Gehalt (iodometrisch): 8,07 mmol/g (92 % d. Th.)

**Patentansprüche**

**1.** Verfahren zur Herstellung von Cysteamin-Säureadditionssalzen durch Umsetzung von Aziridin mit einer anorganischen Schwefelverbindung der Oxidationsstufe -2 und mit einem Keton der Formel I

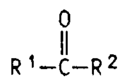 (I),

in der $R^1$ und $R^2$ gleich oder verschieden sind und unabhängig voneinander jeweils $C_1$-$C_6$-Alkyl oder $R^1$ und $R^2$ zusammen $C_4$-$C_6$-Alkylen bedeuten, und anschließender Hydrolyse des als Zwischenstufe gebildeten Thiazolidins der Formel II

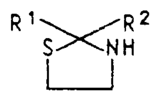 (II),

in der $R^1$ und $R^2$ jeweils die obengenannte Bedeutung besitzen, in Gegenwart einer Säure, dadurch gekennzeichnet, daß man Aziridin und das Keton der Formel I mit Ammoniumhydrogensulfid oder einem Metallhydrogensulfid unter Zugabe einer mittelstarken bis starken Säure bei einer Temperatur von -10 bis +100 °C umsetzt und dabei einen pH-Wert, der größer oder gleich 8,5 ist, einhält.

**2.** Verfahren gemäß Anspruch 1, dadurch gekennzeichnet, daß man die Umsetzung von Aziridin unter Zugabe einer starken anorganischen Säure vornimmt.

**3.** Verfahren gemäß Anspruch 1, dadurch gekennzeichnet, daß man die Umsetzung von Aziridin unter Zugabe von Salzsäure vornimmt.

**Claims**

**1.** A process for the preparation of an addition salt of cysteamine with an acid by reacting aziridine with an inorganic sulfur compound of oxidation state -2 and with a ketone of the formula I

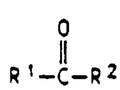

(I)

where $R^1$ and $R^2$ are identical or different and independently of one another are each $C_1$-$C_6$-alkyl, or $R^1$ and $R^2$ together form $C_4$-$C_6$-alkylene, and then subjecting the thiazolidine of the formula II

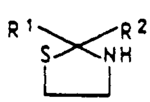

(II)

where $R^1$ and $R^2$ each have the abovementioned meanings, which is formed as an intermediate, to hydrolysis in the presence of an acid, wherein aziridine and the ketone of the formula I are reacted with

ammonium hydrogen sulfide or with a metal hydrogen sulfide with the addition of a moderately strong or strong acid at from -10 to +100° C and a pH which is greater than or equal to 8.5 is maintained.

**2.** A process as claimed in claim 1, wherein the reaction of aziridine is carried out with the addition of a strong inorganic acid.

**3.** A process as claimed in claim 1, wherein the reaction of aziridine is carried out with the addition of hydrochloric acid.

**Revendications**

**1.** Procédé de préparation de sels d'addition d'acide de cystéamine par réaction d'aziridine avec un composé sulfuré inorganique du stade d'oxydation 2 et avec une cétone de formule

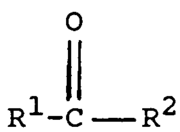

(I)

dans laquelle $R^1$ et $R^2$ sont identiques ou différents et représentent chacun, indépendamment l'un de l'autre, alkyle en $C_1$ - $C_6$ ou $R^1$ et $R^2$, ensemble, alkylène en $C_4$- $C_6$ et ensuite hydrolyse de la thiazolidine de formule II

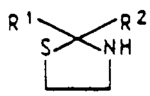

(II)

dans laquelle $R^1$ et $R^2$ ont chacun la signification susindiquée, en présence d'un acide, caractérisé par le fait que l'on fait réagir de l'aziridine et la cétone de formule I avec de l'hydrogénosulfure de métal, avec addition d'un acide de force moyenne à fort, à une température de -10 à + 100 degrés C et on y maintient un pH supérieur ou égal à 8,5.

**2.** Procédé selon la revendication 1, caractérisé par le fait que l'on opère la réaction d'aziridine, sans addition d'un acide inorganique fort.

**3.** Procédé selon la revendication 1, caractérisé par le fait que l'on opère la réaction d'aziridine, sans addition d'acide chlorhydrique.